(19) Europäisches Patentamt
European Patent Office
Office européen des brevets

(11) Veröffentlichungsnummer : **0 439 421 A1**

(12) **EUROPÄISCHE PATENTANMELDUNG**

(21) Anmeldenummer : **91810021.5**

(22) Anmeldetag : **14.01.91**

(51) Int. Cl.[5] : **C07C 317/44,** C07C 309/63, C07D 333/24, C07D 317/60, A01N 41/02

(30) Priorität : **23.01.90 CH 196/90**

(43) Veröffentlichungstag der Anmeldung : **31.07.91 Patentblatt 91/31**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Töpfl, Werner, Dr.**
**Dorneckstrasse 68**
**CH-4143 Dornach (CH)**
Erfinder : **Ackermann, Peter, Dr.**
**Hangelimattweg 113**
**CH-4148 Pfeffingen (CH)**
Erfinder : **Müller, Urs Dr.**
**Drosselstrasse 6**
**CH-4142 Münchenstein (CH)**

(54) **Mikrobizide Mittel.**

(57) Neue 2-Aryl-acrylnitril-Derivate der Formel I,

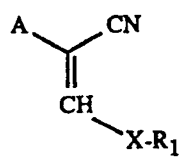

(I),

welche als Gemische (E,Z) oder als reine E- und Z-Isomeren vorliegen, worin $R_1$ unsubstituiertes oder durch Halogen, $C_1$-$C_4$-Alkoxy substituiertes $C_1$-$C_6$-Alkyl oder $C_5$-$C_7$-Cycloalkyl, oder die Reste T, U, U', V oder V'

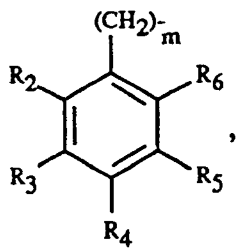

,

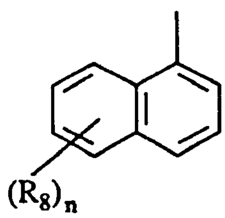

,

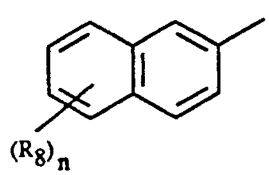

,

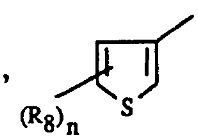

oder

(T) (U) (U') (V)

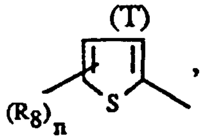

,

(V')

worin m für die Zahlen 0-4, n für die Zahlen 0-2,

$R_3$ zusammen mit $R_2$ oder mit $R_4$ für den Rest 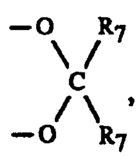 , mit $R_7$ als Wasserstoff, Fluor

oder $C_1$-$C_4$-Alkyl, stehen, oder worin $R_2$-$R_8$ voneinander unabhängig für Wasserstoff, Halogen, Carboxy,

EP 0 439 421 A1

$C_1$-$C_4$-Alkoxycarbonyl, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

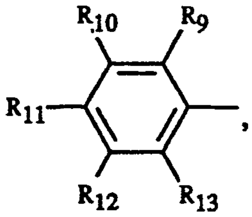

worin $R_{10}$ mit $R_9$ oder $R_{11}$ zusammen für den Rest 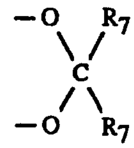 , oder worin $R_9$-$R_{13}$ voneinander unabhängig für Wasserstoff, Halogen, Carboxy, $C_1$-$C_4$-Alkoxycarbonyl,

Nitro, Cyano, die Reste 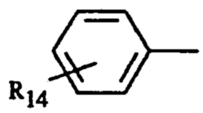 , 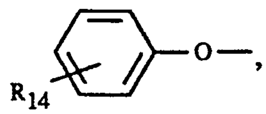 , mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet und
X $-OSO_2-$, -SO- oder $-SO_2-$ bedeutet, wobei wenn X den Rest $-OSO_2-$, $R_{14}$ Wasserstoff oder Chlor und $R_9$, $R_{10}$, $R_{12}$ und $R_{13}$ Wasserstoff bedeuten, T nicht den p-Tolylrest bedeutet und wenn X den Rest $-SO_2-$ bedeutet, $R_1$ nicht den Trifluormethylrest bedeutet.

Die Wirkstoffe der Formel I dienen zur Bekämpfung schädlicher Mikroorganismen, vor allem phytopathogener Pilze. Sie können mit geeigneten Formulierungshilfsstoffen als Mittel eingesetzt werden.

# MIKROBIZIDE MITTEL

Die vorliegende Erfindung betrifft neue substituierte 2-Aryl-acrylnitril-Derivate, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen haben die allgemeine Formel I

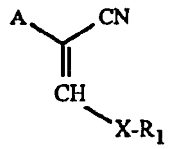 (I),

welche als Gemische (E, Z) oder als reine E- und Z-Isomeren vorliegen, worin $R_1$ unsubstituiertes oder durch Halogen, $C_1$-$C_4$-Alkoxy substituiertes $C_1$-$C_6$-Alkyl oder $C_5$-$C_7$-Cycloalkyl, oder die Reste T, U, U', V oder V'

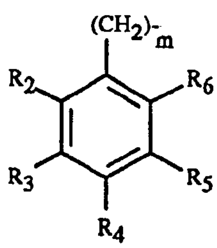 , 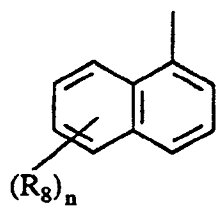 , 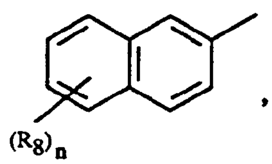 , 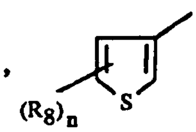 oder

(T) (U) (U') (V)

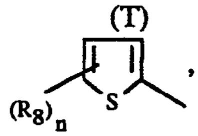 ,

(V')

worin m für die Zahlen 0-4, n für die Zahlen 0-2,

$R_3$ zusammen mit $R_2$ oder mit $R_4$ für den Rest 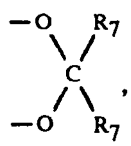 , mit $R_7$ als Wasserstoff, Fluor

oder $C_1$-$C_4$-Alkyl, stehen, oder worin $R_2$-$R_6$ voneinander unabhängig für Wasserstoff, Halogen, Carboxy, $C_1$-$C_4$-Alkoxycarbonyl, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und

$R_8$ für Halogen oder $C_1$-$C_4$-Alkyl stehen, bedeutet,

A den Rest

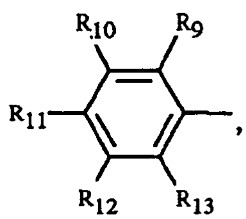 ,

worin $R_{10}$ mit $R_9$ oder $R_{11}$ zusammen für den Rest 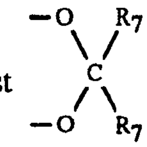, oder worin $R_9$-$R_{13}$ voneinander unabhängig für Wasserstoff, Halogen, Carboxy, $C_1$-$C_4$-Alkoxycarbonyl, Nitro, Cyano, die Reste 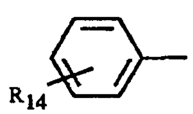, 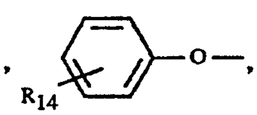, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet und X -$OSO_2$, -SO- oder -$SO_2$- bedeutet, wobei wenn X den Rest -$OSO_2$-, $R_{14}$ Wasserstoff oder Chlor und $R_9$, $R_{10}$, $R_{12}$ und $R_{13}$ Wasserstoff bedeuten, T nicht den p-Tolylrest bedeutet und wenn X den Rest -$SO_2$- bedeutet, $R_1$ nicht den Trifluormethylrest bedeutet.

Unter dem Begriff Alkyl selbst oder als Bestandteil eines anderen Substituenten wie Alkoxy oder Haloalkoxy etc., sind je nach Zahl der angegebenen Kohlenstoffatome beispielsweise die folgenden geradkettigen oder verzweigten Gruppen zu verstehen : Methyl, Ethyl, Propyl, Butyl, Pentyl, Hexyl sowie ihre Isomeren, wie z.B. Isopropyl, Isobutyl, tert.-Butyl, Isoamyl, usw. Halogen und Halo stehen für Fluor, Chlor, Brom oder Jod. Haloalkoxy steht somit für einen einfach bis perhalogenierten Alkoxyrest, wie z.B. $OCF_3$, $OCHCl_2$, $OCH_2F$, $OCCl_3$, $OCH_2Cl$, $OCHF_2$, $OCHFCH_3$, $OCH_2CH_2Br$, $OC_2Cl_5$, $OCH_2Br$, OCHBrCl. $C_5$-$C_7$-Cycloalkyl steht wahlweise für Cyclopentyl, Cyclohexyl oder Cycloheptyl.

Die Bezeichnung der "E"- oder "Z"-Isomeren erfolgt in der vorliegenden Anmeldung gemäss "Nomenclature of Organic Chemistry, Part E". [Pure and Appl. Chem. 45, 11 (1976)].

Die Verbindungen der Formel I sind bei Raumtemperatur stabil. Sie lassen sich auf dem Agrarsektor oder verwandten Gebieten präventiv und kurativ zur Bekämpfung von pflanzenschädigenden Mikroroganismen einsetzen. Die erfindungsgemässen Wirkstoffe der Formel I zeichnen sich in weiten Anwendungskonzentrationen durch eine sehr gute fungizide Wirkung und problemlose Anwendung aus.

Von den Verbindungen der Formel I sind aufgrund ihrer ausgeprägten mikrobiziden Wirkung jene bevorzugt, bei welchen
X den Rest -$OSO_2$- bedeutet.

Diese Verbindungen entsprechen der Formel Ia

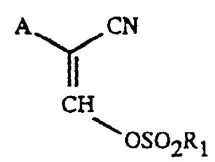
(Ia).

Von den Verbindungen der Formel Ia sind jene insbesondere bevorzugt, bei welchen $R_1$ unsubstituiertes oder durch Halogen substituiertes $C_1$-$C_4$-Alkyl oder $C_5$-$C_6$-Cycloalkyl, oder
die Reste T, U, U', V oder V'

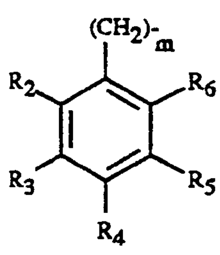, 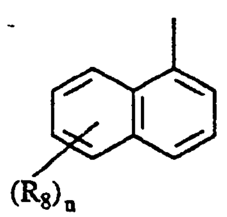, 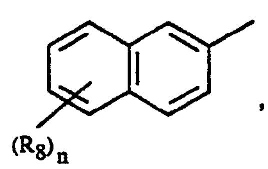, 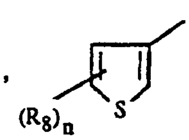

(T) (U) (U') (V)

oder

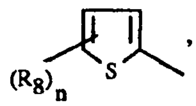

(V')

worin m und n für die Zahlen 0-2,

$R_3$ zusammen mit $R_2$ oder mit $R_4$ für den Rest 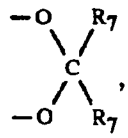 , mit $R_7$ als Wasserstoff, Fluor stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und $R_8$ für Halogen oder $C_1$-$C_4$-Alkyl stehen, bedeutet,
A den Rest

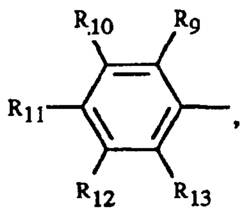

worin $R_{10}$ mit $R_9$ oder $R_{11}$ zusammen für den Rest 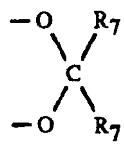 , oder worin $R_9$-$R_{13}$ voneinander unabhängig für Wasserstoff, Halogen, Nitro, Cyano, die Reste 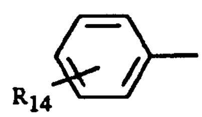

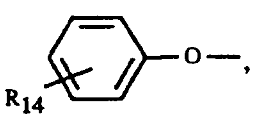 , mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V'bedeutet.

Unter den letztgenannten Verbindungen sind folgende Einzelverbindungen hervorzuheben :

E- und Z-2-Phenyl-3-methylsulfonyloxy-acrylnitril,
E- und Z-2-(4-Chlorphenyl)-3-methylsulfonyloxy-acrylnitril,
E- und Z-2-(2-Tolyl)-3-methylsulfonyloxy-acrylnitril,
E- und Z-2-(3-Tolyl)-3-methylsulfonyloxy-acrylnitril und
Z-2-(3-Tolyl)-3-methylsulfonyloxy-acrylnitril.

Von den Verbindungen der Formel I sind auch jene bevorzugt, bei welchen $R_1$ unsubstituiertes oder durch Halogen substituiertes $C_1$-$C_4$-Alkyl oder $C_5$-$C_6$-Cycloalkyl,
oder
die Reste T, U, U', V oder V'

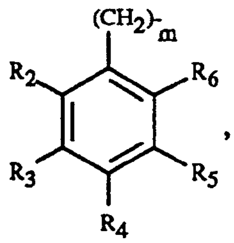, 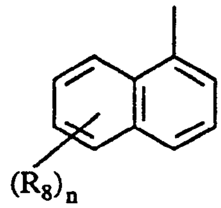, 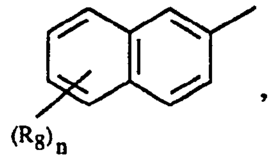, 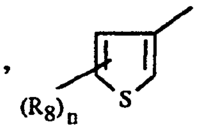 oder

(T) (U) (U') (V)

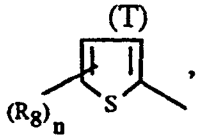,

(V')

worin m und n für die Zahlen 0-2,

$R_3$ zusammen mit $R_2$ oder mit $R_4$ für den Rest 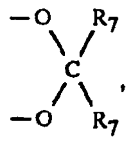, mit $R_7$ als Wasserstoff oder Fluor stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und

$R_8$ für Halogen oder $C_1$-$C_4$-Alkyl stehen, bedeutet,

A den Rest

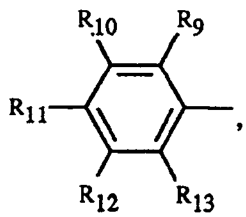,

worin $R_{10}$ mit $R_9$ oder $R_{11}$ zusammen für den Rest 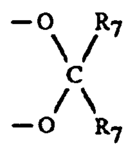, oder worin $R_9$-$R_{13}$ voneinander unabhängig für Wasserstoff, Halogen, Nitro, Cyano, die Reste 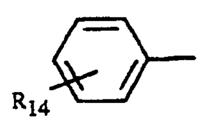, 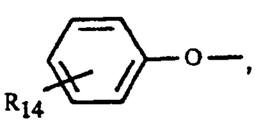, mit $R_{14}$ als Wasserstoff, Halogen oder $C_1$-$C_4$-Alkyl oder für die unsubstituierten oder durch Halogen substituierten reste $C_1$-$C_4$-Alkyl, $C_1$-$C_4$-Alkoxy oder $C_1$-$C_4$-Alkylthio stehen, oder die Reste U, U', V und V' bedeutet und X den Rest -$SOR_1$ bedeutet. Diese Verbindungen entsprechen der Formel Ib

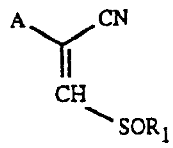

(Ib).

Unter den Verbindungen der Formel Ib sind das E- und Z-2-Phenyl-3-phenylsulfinyl-acrylnitril und das E- und Z-2-Phenyl-3-methylsulfinyl-acrylnitril insbesondere bevorzugt.

Von den Verbindunben der Formel I sind aufgrund ihrer ausgeprägten mikrobiziden Wirkung auch jene bevorzugt, bei welchen

X den Rest $-SO_2-$ bedeutet. Diese Verbindungen entsprechen der Formel Ic

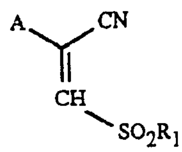

(Ic).

Von dieser Gruppe sind jene Verbindungen insbesondere bevorzugt, bei welchen

$R_1$ unsubstituiertes oder durch Halogen substituiertes $C_1$-$C_4$-Alkyl oder $C_5$-$C_6$-Cycloalkyl,

oder

die Reste T, U, U', V oder V'

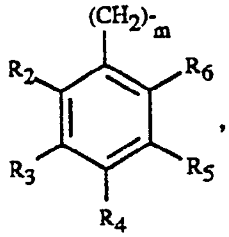 , 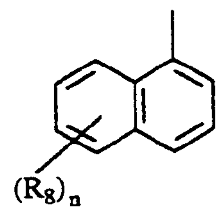 , 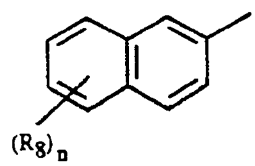 , 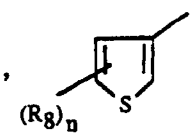

(T) (U) (U') (V)

oder

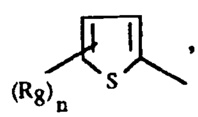 ,

(V')

worin m und n für die Zahlen 0-2,

$R_3$ zusammen mit $R_2$ oder mit $R_4$ für den Rest 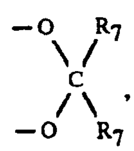 , mit $R_7$ als Wasserstoff oder Fluor stehen, oder worin $R_2$-$R_6$ voneinander unabhänbig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und

$R_8$ für Halogen oder $C_1$-$C_4$-Alkyl stehen, bedeutet,

A den Rest

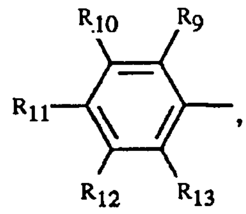

worin $R_{10}$ mit $R_9$ oder $R_{11}$ zusammen für den Rest 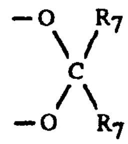 , oder worin $R_9$-$R_{13}$ voneinander unabhängig für Wasserstoff, Halogen, Nitro, Cyano, die Reste 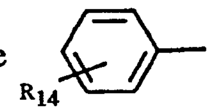

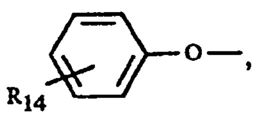, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet und X den Rest $-SO_2-$ bedeutet.

Von dieser Gruppe von Verbindungen sind aufgrund ihrer Aktivität folgende Einzelverbindungen hervorzuheben :

E- und Z-2-Phenyl-3-phenylsulfonyl-acrylnitril,

E- und Z-2-Phenyl-3-(4-tolyl)-sulfonyl-acrylnitril und

Z-2-Phenyl-4-(2,4-difluorphenyl)-sulfonyl-acrylnitril.

Die Herstellung der Verbindungen der Formel Ia kann erfolgen, indem man

$a_1$) ein Nitril der Formel II mit einer Base der Formel III und mit einem Ameisensäureester der Formel IV zu dem Enolat der Formel V umsetzt und letzteres mit einem Sulfochlorid der Formel VI acyliert

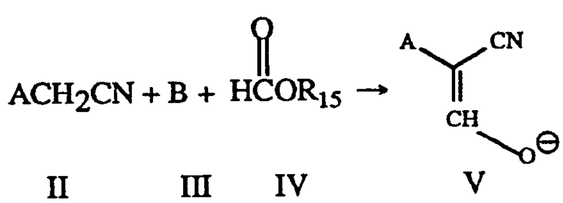

II III IV V

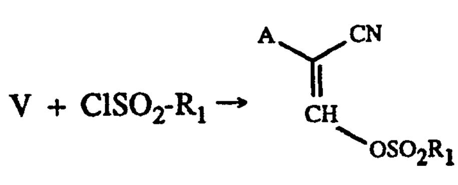

VI Ia

wobei $R_{15}$ $C_1$-$C_4$-Alkyl bedeutet, B eine Base $M^{\oplus}X^{\ominus}$, mit $M^{\oplus}$ für ein Alkali- oder Erdalkalikation und mit $X^{\ominus}$ für ein Alkoholat- oder Hydrid-Anion oder für eine Aminbase, bedeutet und die restlichen Symbole die vorhin angegebenen Bedeutungen haben, oder

$a_2$) eine Verbindung der Formel Va in Gegenwart eines säurebindenden Mittels mit einem Sulfochlorid der Formel VI acyliert

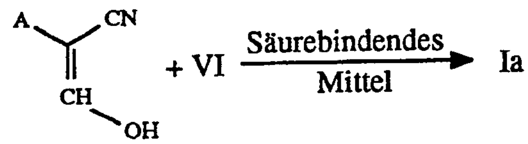


Va

und das Reaktionsprodukt isoliert, wobei A und $R_1$ die obenangegebenen Bedeutungen haben.

Die Herstellung der Verbindungen der Formel Ib kann erfolgen, indem man einen Thioether der Formel VIII mit der äquivalenten Menge eines Oxidationsmittels oxidiert :

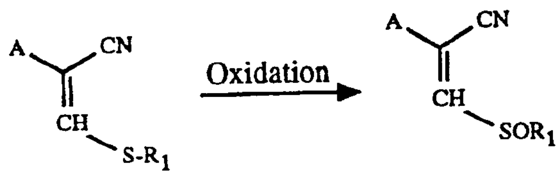


VIII Ib

und das Reaktionsprodukt isoliert.

Die Herstellung der Verbindungen der Formel Ic kann erfolgen, indem man

$c_1$) eine Verbindung der Formel Ia mit einem Sulfinsäuresalz der Formel VII, worin $M^{\oplus}$ und $R_1$ die oben angegebenen Bedeutungen haben

Ia + $M^{\oplus\ominus}O_2S\text{-}R_1$ →

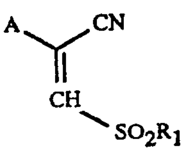

VII Ia

umsetzt oder

$c_2$) ein Sulfoxid der Formel Ib oxidiert

Ib $\xrightarrow{\text{Oxidation}}$ Ic

und das Reaktionsprodukt isoliert.

Die zu den neuen Verbindungen der Formel I führenden Verfahren sind auch Gegenstand der vorliegenden Erfindung.

Das als Ausgangsprodukt der erfindungsgemässen Verfahren eingesetzten Nitrile der Formel II sind entweder bekannt oder können nach bekannten Methoden hergestellt werden. Ihre zu den Verbindungen der Formeln V und Va führende Umsetzung mit Ameisensäureestern ist als Esterkondensation oder als Formylierung zu bezeichnen und ist aus der Literatur bekannt (z.B. Bull. Soc. Chim. France 1961 1144). Diese Umsetzung wird in Gegenwart von organischen Basen, wie beispielsweise Na-Alkoholat, in einem polaren Lösungsmittel, vorzugsweise in einem Alkohol durchgeführt.

Die zu den Enol-Sulfonaten der Formel Ia führende Acylierung der Alkali-Enoläther der Formel V oder der freien Enole der Formel Va mit Sulfochloriden der Formel VI ist eine allgemein bekannte Methode, wobei das Sulfochlorid vorzugsweise in Ueberschuss eingesetzt wird. Ausgehend von Va ist der Einsatz von äquivalenter Menge einer Base zur Neutralisation des als Nebenprodukt entstehenden Chlorwasserstoffs erforderlich. Die Reaktion findet in einem inerten organischen Lösungsmittel, wie beispielsweise Toluol oder Methylenchlorid in der Kälte statt, sie kann durch Zusatz von Stickstoffbasen, wie beispielsweise 4-Dimethylaminopyridin katalysiert werden.

Auch die Sulfide der Formel VIII, welche als Ausgangsprodukte der Verbindungen der Formel Ib und Ic eingesetzt werden, sind aus der Literatur bekannt oder können nach bekannten Verfahren hergestellt werden, Solche Sulfide sind beispielsweise in C.A. 100(2) 14364s, C.A. 89(19) 163199y, C.A. 83(17) 146732a beschrieben. Sie können durch Umsetzung eines Alkalimercaptids mit einem 3-Hydroxyacrylnitril-Derivat, wie beispielsweise 3-Alkoxyacrylnitril hergestellt werden (M. Carion, Bull. Soc. Chim. France, 198-205, 1969). Bevorzugte 3-Hydroxyacrylnitril-Derivate sind die 3-Sulfoxyacrylnitrile der Formel Ia.

Die Herstellung der Sulfoxide der Formel Ib ausgehend von den Sulfiden der Formel VIII erfolgt durch Oxidation mit einem Oxidationsmittel, das in äquivalenter Menge eingesetzt wird. Als Oxidationsmittel eignen sich beispielsweise Peroxide wie Wasserstoffperoxid oder organische Peroxide, vorzugsweise Benzoylperoxid. Die Reaktion verläuft in geeigneten Lösungsmitteln, vorzugsweise in Eisessig vorzugsweise in der Kälte oder bei Raumtemperatur. Detailierte Beschreibung der Sulfoxidherstellung ist in Houben-Weyl : "Methoden der Organischen Chemie", Bd. E11 (1985), Seite 704 ff zu finden.

Auch die Herstellung der Sulfone der Formel Ic kann nach bekannten Methoden erfolgen.

Eine dieser Methoden besteht in der Umsetzung von Enol-Sulfonaten der Formel Ia mit einem Sulfinsäuresalz der Formel VII.

Die Umsetzung verläuft in polaren Lösungsmitteln, wie beispielsweise Alkohole, Amide, Nitrile, Aether und kann durch Kronenäther katalysiert werden.

Dieses Verfahren ist dann besonders bedeutend, wenn in den Verbindungen der Formel Ic $R_1$ einen unsubstituierten oder substituierten Phenyl-, Naphthyl- oder Thienylrest bedeutet.

Die detailierte Beschreibung dieser Reaktion ist zu finden in Houben-Weyl : "Methoden der Organischen Chemie", Bd. E11, (1985) S. 618 ff.

Eine andere Herstellungsmöglichkeit der Sulfone der Formel Ic besteht in der Oxidation der erwähnten Sulfoxide der Formel Ib, oder der Sulfide der Formel VIII. Als Oxidationsmittel eignen sich ebenfalls Peroxide ; die Reaktion verläuft in Analogie zu der Sulfoxid-Herstellung, wobei vorzugsweise das Oxidationsmittel in Ueberschuss unter forcierten Bedingungen eingesetzt wird. Dieses Verfahren ist dann besonders bedeutend, wenn in den Verbindungen der Formel Ic $R_1$ einen unsubstituierten oder substituierten Alkylrest bedeutet, weil andere Methoden in diesem Fall zu kompliziert sind. Die Reaktion ist detailiert beschrieben in Houben-Weyl : "Methoden der Organischen Chemie, Bd E11 (1985) S. 1194 ff zu finden.

Es wurde überraschend festgestellt, dass Verbindungen der Formel I ein für praktische Bedürfnisse sehr günstiges Mikrobizid-Spektrum geben Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam : Ascomyceten (z.B. Venturia, Podosphaera, Erysiphe, Monilinia, Pyrenophora, Uncinula) ; Basidiomyceten (z.B. die Gattungen Hemileia, Rhizoctonia, Puccinia) ; Fungi imperfecti (z.B. Botrytis, Helminthosporium, Fusarium, Septoria, Cercospora, Colletotrichum, Rhynchosporium, Pseudocercosporella und Oomyceten z.B. Phytophthora, Plasmopara, Pythium, Bremia und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung pathogener Mikroorganismen, insbesondere pflanzenschädigender Pilze bzw. die präventive Verhütung eines Befalls an Pflanzen.

Als Zielkulturen für die hierin offenbarten Indikationsgebiete gelten im Rahmen dieser Erfindung z.B. folgende Pflanzenarten : Getreide : (Weizen, Gerste, Roggen, Hafer, Reis, Sorghum und Verwandte) ; Rüben : (Zucker und Futterrüben) ; Kern-, Stein- und Beerenobst : (Äpfel, Birnen, Pflaumen, Pfirsiche, Mandeln, Kirschen, Erdbeeren, Himbeeren und Brombeeren) ; Hülsenfrüchte : (Bohnen, Linsen, Erbsen, Soja) ; Ölkulturen: (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten und Verkapselungen in z.B. polymeren Stoffen in bekannter Weise verarbeitet. Die Anwendungsverfahren werden wie die Art der Mittel den angestrebten Zielen und den gegebenen Verhältnissen entsprechend gewählt. Im Agrarsektor liegen günstige Aufwandmengen im allgemeinen bei 50 g bis 5 kg Aktivsubstanz (AS) je ha ; bevorzugt 100 g bis 2 kg AS/ha, insbesondere bei 150 g bis 800 g.

Als Lösungsmittel können in Frage kommen : Aromatische Kohlenwasserstoffe, bevorzugt die Fraktion $C_8$-$C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:
"Mc Cutcheon's Detergents and Emulsifiers Annual"
BC Publishing Corp., Ringwood New Jersey, 1981 ;
Helmut Stache "Tensid-Taschenbuch" Carl Hanser-Verlag
München/Wien 1981.
M. and J. Ash. "Encyclopedia of Surfactants", Vol. I-III, Chemical Publishing Co., New York, 1980-1981.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Streckmittels und 0 bis 25 %, insbesondere 0,1 bis 25 % eines Tensides.

Wärend als Handelsware eher konzentrierte Mittel bevorzugt werden, verwendet der Endverbraucher in der Regel verdünnte Mittel.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht.

Herstellungsbeispiele

H1. Herstellung des Natriumenolates von 2-Formyl-2-(4-tolyl)-acrylnitril. (Verbindung Nr. 1.1, Tabelle 1)

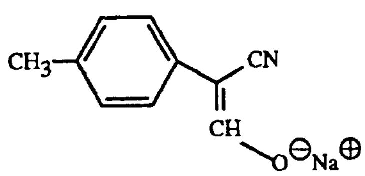

Zu einer Lösung von 660 g 4-Tolylacetonitril, 363 g Ameisensäuremethylester und 400 ml Methanol werden innerhalb von 45 Minuten 918 g einer 30%-igen Natriummethylatlösung in Methanol zugetropft. Bei der Zugabe steigt die Temperatur auf ca. 35°C. Das Reaktionsgemisch wird anschliessend 12 Stunden bei Raumtemperatur gerührt, es bildet sich ein dicker Brei. Dieser wird anschliessend mit 500 ml Aether verdünnt ; der Niederschlag wird abfiltriert, 2-mal mit je 100 ml Aether nachgewaschen und getrocknet. das erhaltene Titelprodukt hat einen Schmelzpunkt von über 230°C.

H2. Herstellung von 2-(4-Tolyl)-3-methansulfonyloxy-acrylnitril (Verbindung Nr. 2.19, Tabelle 2).

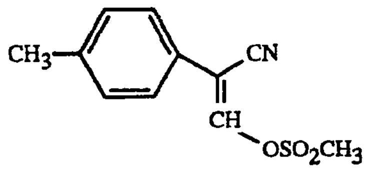

500 g der Verbindung Nr. 1.1 aus Beispiel H1 werden in 3,4 Liter Methylenchlorid suspendiert und auf -5°C abgekühlt. Bei dieser Temperatur versetzt man das Reaktionsgemisch zuerst mit ca. 200 mg 4-Dimethylaminopyridin und anschliessend innerhalb von einer Stunde mit 570 g Methansulfochlorid. Anschliessend lässt man das Gemisch auf Raumtemperatur erwärmen, wobei die immer dicker werdende Suspension während 14 Stunden gerührt wird. Nun giesst man das Reaktionsgut auf 3 Liter Wasser, trennt die danach entstandenen zwei klaren Phasen und wäscht die organische Phase 2-mal mit je 1,5 Liter Sole. Die organische Phase wird abgetrennt, über Magnesiumsulfat getrocknet und unter vermindertem Druck zur Hälfte eingedampft. Die gebildeten Kristalle werden abfiltriert und mit 1 Liter Aether nachgewaschen. Aus der Mutterlauge können weitere, reine Kristallfraktionen gewonnen werden. Man erhält das Titelprodukt mit einem Smp. von 125-126°C. Diese Verbindung enthält zu mehr als 90 % das Z-Isomere.

H3. Herstellung von 2-(4-Tolyl)-3-phenylsulfonyl-acrylnitril

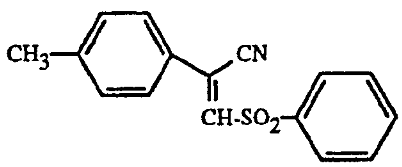

6 g 2-(4-Tolyl)-3-methansulfonyloxy-acrylnitril, gelöst in 45 ml N-Methylpyrrolidon, werden mit 8,3 g Benzolsulfinsäure-Natriumsalz versetzt. Die gelbe Suspension wird über Nacht bei Raumtemperatur gerührt. Anschliessend wird das Reaktionsgut mit 900 ml Wasser und 400 ml Essigester versetzt. Die resultierenden zwei klaren Phasen werden voneinander getrennt. Die organische Phase wird nach Waschen mit 2-mal 300 ml Sole über Magnesiumsulfat getrocknet und unter vermindertem Druck eingedampft. Nach Umkristallisation des festen Rückstands aus Essigester/Hexan wird das Titelprodukt mit einem Smp. von 143-145°C erhalten.

In ähnlicher Weise können folgende Verbindungen hergestellt werden.

Tabelle 1 Verbindungen der Formel

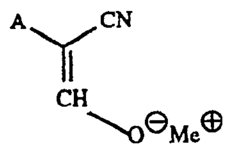

| Verb. Nr. | A | $Me^+$ | Physikalische Eigenschaften |
|---|---|---|---|
| 1.1 | $4\text{-}CH_3\text{-}C_6H_4\text{-}$ | $Na^+$ | Smp. >230°C |

Tabelle 2 Verbindungen der Formel

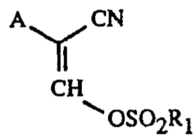

| Verb. Nr. | A | $R_1$ | Isomer | Physikalische Eigenschaften |
|---|---|---|---|---|
| 2.1 | $C_6H_5$- | $CH_3$- | E/Z | Smp.: 92-95°C |
| 2.2 | 3-Thienyl | $CH_3$- | | Smp.: 131-133°C |
| 2.3 | 2-Thienyl | $CH_3$- | | Smp.: 94-96°C |
| 2.4 | 4-Cl-$C_6H_4$- | $CH_3$- | | Smp.: 105-109°C |
| 2.5 | 4-F-$C_6H_4$- | $CH_3$- | | Smp.: 100-102°C |
| 2.6 | 4-$OCH_3$-$C_6H_4$- | $CH_3$- | E/Z | Smp.: 89-90°C |
| 2.7 | 3-$OCH_3$-$C_6H_4$- | $CH_3$- | E/Z | Smp.: 77-80°C |
| 2.8 | $C_6H_5$- | 4-$CH_3$-$C_6H_4$- | E/Z | Smp.: 112-135°C |
| 2.9 | $C_6H_5$- | 2-$NO_2$-$C_6H_4$- | E/Z | Smp.: 119-121°C |
| 2.10 | 2-$OCH_3$-$C_6H_4$- | $CH_3$- | | Smp.: 77-78°C |
| 2.11 | $C_6H_5$- | 2,4-$F_2$-$C_6H_3$- | E/Z | Smp.: 90-127°C |
| 2.12 | $C_6H_5$- | 3-Cl-$C_6H_4$- | | Smp.: 57-60°C |
| 2.13 | $C_6H_5$- | 4-F, 3-Cl-$C_6H_3$- | E/Z | Smp.: 64-74°C |
| 2.14 | $C_6H_5$- | 3,5-$Cl_2$- | E/Z | Smp.: 99-115°C |
| 2.15 | 2-F-$C_6H_4$- | $CH_3$-$C_6H_4$- | | Smp.: 112-135°C |
| 2.16 | 2-Thienyl | 4-Br-$C_6H_4$- | | Smp.: 118-120°C |
| 2.17 | 2,4-$Cl_2$-$C_6H_3$- | 4-Br-$C_6H_4$- | | Smp.: 109-111°C |
| 2.18 | 4-$OC_2H_5$-$C_6H_4$- | $CH_3$- | E/Z | Smp.: 76-78°C |
| 2.19 | 4-$CH_3$-$C_6H_4$- | $CH_3$- | Z | Smp.: 125-127°C |
| 2.20 | 2-$CH_3$-$C_6H_4$- | $CH_3$- | | $n_D^{28}$ = 1,5432 |
| 2.21 | $C_6H_5$- | 3,4-$(OCH_3)_2$-$C_6H_3$- | E/Z | Smp.: 78-87°C |
| 2.22 | $C_6H_5$- | 3,4-$Cl_2$-$C_6H_3$- | E/Z | Smp.: 85-95°C |
| 2.23 | $C_6H_5$- | 2-Cl, 4-F-$C_6H_3$- | | Smp.: 78-79°C |
| 2.24 | $C_6H_5$- | 4-$NO_2$-$C_6H_4$- | | Smp.: 161-162°C |
| 2.25 | $C_6H_5$- | 4-Cl-$C_6H_4$- | E/Z | Smp.: 142-153°C |
| 2.26 | $C_6H_5$- | 2-Cl-4-CN-$C_6H_4$- | | Smp.: 144-145°C |
| 2.27 | $C_6H_5$- | 2,4-$Cl_2$-$C_6H_3$- | | Smp.: 171-174°C |
| 2.28 | 3-$CH_3$-$C_6H_4$- | $CH_3$- | Z | Smp.: 98°C |

Tabelle 2 (Fortsetzung)

| Verb. Nr. | A | $R_1$ | Isomer | Physikalische Eigenschaften |
|---|---|---|---|---|
| 2.29 | 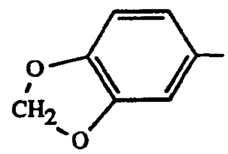 | $CH_3$- | E/Z | Smp.: 106-108°C |
| 2.30 | 2-Br-$C_6H_4$- | $CH_3$- | Z | Smp.: 120-121°C |
| 2.31 | 4-$OCH_3$-$C_6H_4$- | $CF_3$- | E | $n_D^{25}$ = 1,5099 |
| 2.32 | 4-$OCH_3$-$C_6H_4$- | $CF_3$- | Z | Smp.: 58-60°C |
| 2.33 | (4-$C_4H_5$)-$C_6H_4$- | $CH_3$- | E/Z | Smp.: 140-143°C |
| 2.34 | 2-F, 6-Cl-$C_6H_3$- | $CH_3$- | E/Z | Smp.: 74-76°C |
| 2.35 | β-Naphthyl | $CH_3$- | | Smp.: 141-143°C |
| 2.36 | 2,6-$Cl_2$-$C_6H_3$- | $CH_3$- | Z | fest |
| 2.37 | 3,4-$(OCH_3)_2C_6H_3$- | $CH_3$- | | fest |
| 2.38 | α-Naphthyl- | $CH_3$- | | Smp.: 138-140°C |
| 2.39 | 4-Br-$C_6H_4$- | $CH_3$- | | Smp.: 123-125°C |
| 2.40 | 3-$CF_3$-$C_6H_4$- | $CH_3$- | | Smp.: 97-99°C |
| 2.41 | $C_6H_5$- | $CF_3$- | | |
| 2.42 | 3-$CH_3$-$C_6H_4$- | $CF_3$- | | $n_D^{26}$ = 1,5268 |
| 2.43 | 3-$CH_3$-$C_6H_4$- | $C_3H_7$- | | Smp.: 25-30°C |
| 2.44 | 3-$CH_3$-$C_6H_4$- | $C_4H_9$- | | $n_D^{26}$ = 1,5349 |
| 2.45 | 3-$CH_3$-$C_6H_4$- | $C_2H_5$- | | $n_D^{24}$ = 1,5460 |
| 2.46 | 3-$CH_3$-$C_6H_4$- | $CH_2$-$CF_3$- | | |
| 2.47 | 3-$CH_3$-$C_6H_4$- | 2-Thienyl | | |
| 2.48 | 3-$CH_3$-$C_6H_4$- | $CCl_3$- | | |
| 2.49 | 3-$CH_3$-$C_6H_4$- | i-$C_3H_7$- | | |
| 2.50 | 3-$CH_3$-$C_6H_4$- | β-Naphthyl | | |

Tabelle 3 Verbindungen der Formel

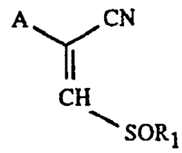

| Verb. Nr. | A | $R_1$ | Isomer | Physikalische Eigenschaften |
|---|---|---|---|---|
| 3.1 | $C_6H_5$- | $CH_3$- | E/Z | Smp.: 91-95°C |
| 3.2 | $C_6H_5$- | $C_6H_5$-$CH_2$- | Z | Smp.: 106°C |
| 3.3 | $C_6H_5$- | $C_6H_5$-$CH_2$- | E | Smp.: 110°C |
| 3.4 | $C_6H_5$- | $C_6H_5$- | E/Z | Smp.: 90-105°C |
| 3.5 | $C_6H_5$- | n-$C_4H_9$ | E | Smp.: 66-68°C |
| 3.6 | $C_6H_5$- | n-$C_4H_9$ | Z | Oel |
| 3.7 | $C_6H_5$- | i-$C_3H_7$ | Z | Smp.: 75-78°C |
| 3.8 | $C_6H_5$- | 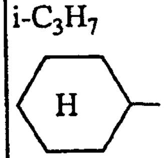 | E | Smp.: 106-108°C |
| 3.9 | $C_6H_5$- | 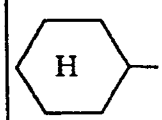 | | Smp.: 57-60°C |
| 3.10 | $C_6H_5$- | $(CH_3)C$- | E/Z | Smp.: 119-121°C |
| 3.11 | $C_6H_5$- | 4-Cl-$C_6H_4$- | E | Smp.: 115-116°C |
| 3.12 | $C_6H_5$- | 4-Cl-$C_6H_4$- | Z | Smp.: 132-133°C |
| 3.13 | $C_6H_5$- | -$CH_2COOCH_3$ | Z | Smp.: 95-97°C |
| 3.14 | $C_6H_5$- | $ClCH_2CH_2CH_2$- | E | Smp.: 72-73°C |
| 3.15 | $C_6H_5$- | $ClCH_2CH_2CH_2$- | Z | Smp.: 65-66°C |

Tabelle 4 Verbindungen der Formel

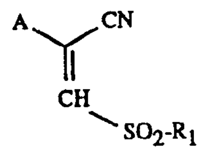

| Verb. Nr. | A | $R_1$ | Isomer | Physikalische Eigenschaften |
|---|---|---|---|---|
| 4.1 | $C_6H_5$- | $C_6H_5$- | E/Z | Smp.: 132-134°C |
| 4.2 | 4-Cl-$C_6H_4$- | $C_6H_5$- | E/Z | Smp.: 84-89°C |
| 4.3 | 4-F-$C_6H_4$- | $C_6H_5$- | E/Z | Smp.: 72-78°C |
| 4.4 | 3-$CF_3$-$C_6H_4$- | $C_6H_5$- | E/Z | Smp.: 116-121°C |
| 4.5 | 2,4-$Cl_2$-$C_6H_3$- | $C_6H_5$- | E/Z | Smp.: 160-161°C |
| 4.6 | $C_6H_5$- | 4-$CH_3$-$C_6H_4$- | E/Z | Smp.: 89-103°C |
| 4.7 | $C_6H_5$- | 4-$CH_3$-$C_6H_4$- | E | Smp.: 96-100°C |
| 4.8 | $C_6H_5$- | 4-$CH_3$-$C_6H_4$- | Z | Smp.: 133-136°C |
| 4.9 | $C_6H_5$- | $C_6H_5$- | E | Smp.: 103-104°C |
| 4.10 | $C_6H_5$- | $C_6H_5$- | Z | Smp.: 124-127°C |
| 4.11 | 3-$CF_3$-$C_6H_4$- | 2-(COOH)-$C_6H_4$- | E/Z | Smp.: 220-224°C (Zers.) |
| 412 | 2-Thienyl | $C_6H_5$- | E/Z | Smp.: 183-185°C (Zers.) |
| 4.13 | 2,4-$Cl_2$-$C_6H_3$- | 2-(COOH)-$C_6H_4$- | E/Z | Smp.: 225-226°C (Zers.) |
| 4.14 | 4-$OCH_3$-$C_6H_4$- | $C_6H_5$- | E/Z | Smp.: 112-114°C |
| 4.15 | 4-$OCH_3$-$C_6H_4$- | 4-$CH_3$-$C_6H_4$- | Z | Smp.: 96-97°C |
| 4.16 | 4-$OCH_3$-$C_6H_4$- | 4-$CH_3$-$C_6H_4$- | E | Smp.: 94-96°C |
| 4.17 | 4-$OC_2H_5$-$C_6H_5$- | 4-$CH_3$-$C_6H_4$- | E | Smp.: 81-83°C |
| 4.18 | 4-$OC_2H_5$-$C_6H_4$- | 4-$CH_3$-$C_6H_4$- | Z | Smp.: 118-120°C |
| 4.19 | 4-$OC_2H_5$-$C_6H_4$- | $C_6H_5$- | E | Smp.: 77-80°C |
| 4.20 | 4-$OC_2H_5$-$C_6H_4$- | $C_6H_5$- | Z | Smp. 116-118°C |
| 4.21 | $C_6H_5$- | 3,5-$Cl_2$-$C_6H_3$- | E | Smp.: 105-106°C |
| 4.22 | $C_6H_5$- | 3,5-$Cl_2$-$C_6H_3$- | Z | Smp.: 165°C |
| 4.23 | $C_6H_5$- | 3-Cl-$C_6H_4$- | Z | Smp.: 144-146°C |
| 4.24 | $C_6H_5$- | 4-F, 3-Cl-$C_6H_3$- | Z | Smp.: 114-117°C |
| 4.25 | $C_6H_5$- | $CH_3$- | E/Z | Smp.: 122-124°C |
| 4.26 | $C_6H_5$- | 2,4-$F_2$-$C_6H_3$- | Z | Smp.: 107-110°C |
| 4.27 | 3-$OCH_3$-$C_6H_4$- | $C_6H_5$- | Z | Smp.: 126-127°C |
| 4.28 | 3-$OCH_3$-$C_6H_4$- | 4-$CH_3$-$C_6H_4$- | E/Z | Smp.: 163-166°C |

Tabelle 4 (Fortsetzung)

| Verb. Nr. | A | $R_1$ | Isomer | Physikalische Eigenschaften |
|---|---|---|---|---|
| 4.29 | $4\text{-}CH_3\text{-}C_6H_4\text{-}$ | $C_6H_5\text{-}$ | E/Z | Smp.: 143-145°C |
| 4.30 | $2\text{-}F\text{-}C_6H_4\text{-}$ | $4\text{-}CH_3\text{-}C_6H_4\text{-}$ | E/Z | Smp.: 90-91°C |
| 4.31 | $4\text{-}F\text{-}C_6H_4\text{-}$ | $C_6H_5\text{-}$ | Z | Smp.: 110°C |
| 4.32 | $4\text{-}CH_3\text{-}C_6H_4\text{-}$ | $4\text{-}CH_3\text{-}C_6H_4\text{-}$ | E/Z | Smp.: 157°C |
| 4.33 | $2\text{-}OCH_3\text{-}C_6H_4\text{-}$ | $C_6H_5\text{-}$ | Z | Smp.: 97-100°C |
| 4.34 | $3\text{-}CH_3\text{-}C_6H_4\text{-}$ | $4\text{-}CH_3\text{-}C_6H_4\text{-}$ | Z | Smp.: 113°C |
| 4.35 | $2\text{-}OCH_3\text{-}C_6H_4\text{-}$ | $4\text{-}CH_3\text{-}C_6H_4\text{-}$ | Z | Smp.: 91°C |
| 4.36 | $3\text{-}CH_3\text{-}C_6H_4\text{-}$ | $C_6H_5\text{-}$ | Z | Smp.: 106°C |
| 4.37 | $C_6H_5\text{-}$ | $2,4\text{-}F_2\text{-}C_6H_3\text{-}$ | E | Smp.: 88-89°C |
| 4.38 | $C_6H_5\text{-}$ | $CH_2\text{-}C_6H_5\text{-}$ | | Smp.: 111-112°C |
| 4.39 | $C_6H_5\text{-}$ | $4\text{-}Cl\text{-}C_6H_5\text{-}$ | | Smp.: 98-100°C |
| 4.40 | $C_6H_5\text{-}$ | $2\text{-}NO_2\text{-}C_6H_5\text{-}$ | | Smp.: 200°C |
| 4.41 | $C_6H_5\text{-}$ | $2,4\text{-}Cl_2\text{-}C_6H_3\text{-}$ | | Smp.: 126-127°C |
| 4.42 | $C_6H_5\text{-}$ | $2\text{-}Cl\text{-}4\text{-}CN\text{-}C_6H_3\text{-}$ | | Smp.: 184-185°C |
| 4.43 | $C_6H_5\text{-}$ | $3,4(\text{-}OCH_3)\text{-}C_6H_3\text{-}$ | | Smp.: 112-113°C |
| 4.44 | $C_6H_5\text{-}$ | $3,4\text{-}Cl_2\text{-}C_6H_3\text{-}$ | | Smp.: 168-169°C |
| 4.45 | $C_6H_5\text{-}$ | $2\text{-}Cl\text{-}4F\text{-}C_6H_3\text{-}$ | Z | Smp.: 140-141°C |
| 4.46 | $C_6H_5\text{-}$ | $2\text{-}Cl\text{-}C_6H_4\text{-}$ | | Smp. 159°C |
| 4.47 | $2\text{-}CH_3\text{-}C_6H_5\text{-}$ | $4\text{-}CH_3\text{-}C_6H_4\text{-}$ | Z | Harz |
| 4.48 | $2\text{-}CH_3\text{-}C_6H_5\text{-}$ | $C_6H_5\text{-}$ | Z | Smp.: 87-89°C |
| 4.49 | 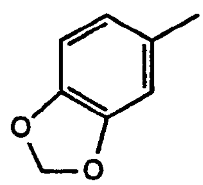 | $C_6H_5\text{-}$ | | Smp.: 178-181°C |
| 4.50 | $3\text{-}Br\text{-}C_6H_4\text{-}$ | $4\text{-}CH_3\text{-}C_6H_4\text{-}$ | | Smp.: 131-132°C |

Tabelle 4 (Fortsetzung)

| Verb. Nr. | A | $R_1$ | Isomer | Physikalische Eigenschaften |
|---|---|---|---|---|
| 4.51 | $C_6H_5$- | n-Butyl- | E | Oel |
| 4.52 | $C_6H_5$- | -n-Butyl- | Z | Smp. 63-64°C |
| 4.53 | 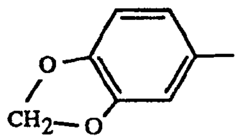 | 4-$CH_3$-$C_6H_4$- | Z | Smp.: 224-226°C |
| 4.54 | $C_6H_5$- | i-Propyl | E | Smp.: 113-114°C |
| 4.55 | $C_6H_5$- | i-Propyl | Z | Smp.: 78-81°C |
| 4.56 | $C_6H_5$- | -t-Butyl- | E/Z | Smp.: 121-160°C |
| 4.57 | $C_6H_5$- | Cyclohexyl- | E | Smp.: 97-98°C |
| 4.58 | $C_6H_5$- | Cyclohexyl- | Z | Smp.: 113-115°C |
| 4.59 | $C_6H_5$- | $CH_2$-$C_6H_4$-4-Cl- | E | Smp.: 100-101°C |
| 4.60 | $C_6H_5$- | $CH_2$-$C_6H_4$-4-Cl- | Z | Smp.: 173-174°C |
| 4.61 | 4-($C_6H_5$)-$C_6H_5$- | 4-$CH_3$-$C_6H_4$- | Z | Smp.: 160-165°C |
| 4.62 | 2,6-$Cl_2$-$C_6H_5$- | 4-$CH_3$-$C_6H_4$- | | Smp.: 142°C |
| 4.63 | 4-($C_6H_5$)-$C_6H_4$- | $C_6H_5$- | Z | fest |
| 4.64 | 2,6-$Cl_2$-$C_6H_9$- | $C_6H_5$- | | Smp.: 120-121°C |
| 4.65 | $C_6H_5$- | $ClCH_2CH_2CH_2$- | E | Oel |
| 4.66 | $C_6H_5$- | $ClCH_2CH_2CH_2$- | Z | Smp.: 65-68°C |
| 4.67 | α-Naphthyl- | $C_6H_5$- | | Smp.: 93-94°C |
| 4.68 | α-Naphthyl- | 4-$CH_3$-$C_6H_5$- | | Smp.: 133-135°C |
| 4.69 | β-Naphthyl- | 4-$CH_3$-$C_6H_5$- | | Smp.: 160-161°C |
| 4.70 | 3,4-$(OCH_3)_2$-$C_6H_3$- | $C_6H_5$- | E | Smp.: 114-116°C |
| 4.71 | 3,4-$(OCH_3)_2$-$C_6H_3$- | $C_6H_5$- | Z | Smp.: 150-152°C |
| 4.72 | 3,4-$(OCH_3)_2$-$C_6H_3$- | 4-$CH_3$-$C_6H_4$- | E | Smp.: 120-122°C |
| 4.73 | 3,4-$(OCH_3)_2$-$C_6H_3$- | 4-$CH_3$-$C_6H_4$- | Z | Smp.: 151-152°C |
| 4.74 | β-Naphthyl- | $C_6H_5$- | Z | Smp.: 217°C (Zers.) |
| 4.75 | 4-Br-$C_6H_4$- | $C_6H_5$- | | Smp.: 153-154°C |
| 4.76 | 4-Br-$C_6H_4$- | 4-$CH_3$-$C_6H_4$- | | Smp.: 148-150°C |

Formulierungsbeispiele für Wirkstoffe der Formeln Ia, Ib und Ic (% = Gewichtsprozent)

| F1. Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| F2. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F3. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| F4. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F5. Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

F6. Umhüllungs-Granulat

| | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

Biologische Beispiele :

Beispiel B1 : Wirkung gegen Cercospora arachidicola auf Erdnusspflanzen

a) Residual-protektive Wirkung

10-15 cm hohe Erdnusspflanzen werden mit einer aus Spritzpulver der Wirksubstanz hergestellten Spritzbrühe (200 ppM Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) Systemische Wirkung

Zu 10-15 cm hohen Erdnusspflanzen wird eine aus Spritzpulver des Wirkstoffes hergestellte Spritzbrühe gegossen (20 ppM Aktivsubstanz bezogen auf das Erdvolumen). Nach 48 Stunden werden die behandelten Pflanzen mit einer Konidiensuspension des Pilzes infiziert und 72 Stunden bei ca. 21°C und hoher Luftfeuchigkeit inkubiert. Anschliessend werden die Pflanzen im Gewächshaus aufgestellt, und nach 11 Tagen wird der Pilzbefall beurteilt.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhinderten die Verbindungen Nr. 2.4, 2.19, 2.28, 4.6 und 4.26 in obigen Versuchen das Auftreten von Flecken fast vollständig (0-10 %).

Beispiel B2 : Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (200 ppM Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Verbindungen aus den Tabellen bewirkten eine deutliche Hemmung des Krankheitsbefalls. So hemmten die Verbindungen 2.4, 2.19, 2.28, 4.6 und 4.26 den Pilzbefall fast vollständig (0.5 %). Unbehandelte aber infizierte Triebe zeigten einen 100%igen Venturia-Befall.

Beispiel B3 : Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (200 ppM Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgt die Beurteilung des Pilzbefalls.

Der Botrytis-Befall unbehandelter aber infizierter Bohnenpflanzen betrub 100 %. Der Befall nach Behandlung mit einer der Verbindungen der Formel I betrug <20 % ; bei Behandlung mit den Verbindungen Nr. 2.28 und 4.26 trat kein Befall auf (0-5 %).

Beispiel B4 : Wirkung gegen Rhizoctonia solani (Bodenpilz auf Reispflanzen)

a) Protektiv-lokale Bodenapplikation

12 Tage alte Reispflanzen werden mit einer aus einer Formulierung des Wirkstoffes hergestellten Spritzbrühe (20 ppM Aktivsubstanz), ohne oberirdische Pflanzenteile zu benetzen, angegossen. Zur Infizierung der behandelten Pflanzen wird eine Suspension von Myzel und Sklerotien von R. solani auf die Bodenoberfläche gegeben. Nach 6-tägiger Inkubation bei 27°C (Tag), bzw. 23° (Nacht) und 100 % rel. Luftfeuchtigkeit (Feuchtkasten) in der Klimakammer wird der Pilzbefall auf Blattscheide, Blättern und Stengel beurteilt.

b) Protektiv-lokale Blattapplikation

12 Tage alte Reispflanzen werden mit einer aus einer Formulierung der Wirkstoffe hergestellten Spritzbrühe besprüht. Einen Tag später werden die behandelten Pflanzen mit 20 ppM einer Suspension von Myzel und Sklerotien von R. solani infiziert. Nach 6-tägiger Inkubation bei 27°C (Tag), bzw. 23°C (Nacht) und 100 % rel. Luftfeuchtigkeit (Feuchtkasten) in der Klimakammer wird der Pilzbefall auf Blattscheide, Blättern und Stengel beurteilt.

Verbindungen aus den Tabellen zeigten gute Wirksamkeit durch Hemmung des Rhizoctonia-Befalls. So hemmte z.B. die Verbindungn 2.28 den Pilzbefall bis auf 0 bis 5 %. Unbehandelte jedoch infizierte Kontrollpflanzen wiesen dagegen einen Befall von 100 % auf.

Beispiel B5 : Wirkung gegen Botrytis cinerea an Apfelfrüchten, Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgepfropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die anbefaulten Verletzungsstellen gezählt und daraus die fungizide Wirkung der Testsubstanz abgeleitet.

Verbindungen aus den Tabellen 2 bis 4 zeigen gegen Botrytis gute Wirksamkeit (Befall : weniger als 20 %). So reduzierten z.B. die Verbindungen Nr. 2.4, 4.6 und 4.26 den Botrytis-Befall auf 0 bis 10%. Unbehandelte aber infizierte Kontrollpflanzen wiesen dagegen einen Botrytis-Befall von 100 % auf.

Beispiel B6 : Wirkung gegen Plasmopara viticola auf Reben

a) Residual-protektive Wirkung

Im 4-5 Blattstadium wurden Rebensämlinge mit einer aus Spritzpulver des Wirkstoffes hergestellten Spritzbrühe (200 ppM Aktivsubstanz) besprüht. Nach 24 Stunden wurden die behandelten Pflanzen mit einer Sporangiensuspension des Pilzes infiziert. Nach einer Inkubation während 6 Tagen bei 95-100 % relativer Luftfeuchtigkeit und 20°C wurde der Pilzbefall beurteilt.

b) Residual-kurative Wirkung

Im 4-5 Blattstadium wurden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C wurden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus den Tabellen 2 bis 4 zeigten geben Plasmopara viticola auf Reben eine sehr gute fungizide Wirkung, insbesondere die Wirkstoffe Nr. 2.4, 2.19, 2.28 4.6 und 4.26, welche eine vollständige Unterdrückung des Pilzbefalls (Restbefall 0 bis 5 %) bewirkten.

Beispiel B7 : Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen 2 bis 4 zeigten gegen den Phytophthora-Pilz eine gute Schutzwirkung. So reduzierten z.B. die Verbindungen 2.4, 2.28, 4.6 und 4.26 den Pilzbefall auf 0 bis 20 %. Unbehandelte jedoch infizierte Kontrollpflanzen wiesen dagegen einen 100%igen Phytophthora-Befall auf.

Beispiel B8 : Wirkung gegen Pythium debaryanum an Zea mays

a) Bodenapplikation

Der Pilz wurde auf Karottenschnitzel-Nährlösung kultiviert und einer Erde-Sand-Mischung beigegeben. Die so infizierte Erde wurde in Blumentöpfe abgefüllt und mit Maissamen besät. Gleich nach der Aussaat wurden die als Spritzpulver formulierten Versuchspräparate als wässrige Suspensionen über Erde gegossen (20 ppm Wirkstoff bezogen auf das Erdvolumen). Die Töpfe wurden darauf während 2-3 Wochen im Gewächshaus bei ca. 20°C aufgestellt. Die Erde wurde dabei durch leichtes Ueberbrausen stets gleichmässig feucht gehalten. Bei der Auswertung der Tests wurde der Auflauf der Maispflanzen sowie der Anteil gesunder und kranker Pflanzen bestimmt.

b) Wirkung nach Beizapplikation

Der Pilz wurde auf Karottenschnitzel-Nährlösung kultiviert und einer Erde-Sand-Mischung beigegeben. Die so infizierte Erde wurd in Erdschalen abgefüllt und mit Maissamen besät, die mit den als Beizpulver formulierten Versuchspräparaten gebeizt worden waren (0,06 % Wirkstoff).

Die besäten Töpfe wurden während 2-3 Wochen im Gewächshaus bei ca. 20°C aufgestellt. Die Erde wurde dabei durch leichtes Ueberbrausen stets gleichmässig feucht gehalten. Bei der Auswertung wurd der Auflauf der Maispflanzen bestimmt.

Nach der Behandlung mit dem Wirkstoff 2.1 liefen, sowohl unter den Testbedingungen a) wie b) mehr als 85 % Maispflanzen auf und hatten ein gesundes Aussehen. Bei der unbehandelten Kontrolle liefen weniger als 20 % Pflanzen mit zum Teil kränklichem Aussehen auf.

## Patentansprüche

1. 2-Aryl-acrylnitril-Derivate der Formel I,

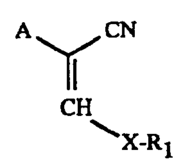

(I),

welche als Gemische (E,Z) oder als reine E- und Z-Isomeren vorliegen, worin $R_1$ unsubstituiertes oder durch Halogen, $C_1$-$C_4$-Alkoxy substituiertes $C_1$-$C_6$-Alkyl oder $C_5$-$C_7$-Cycloalkyl, oder die Reste T, U, U', V oder V'

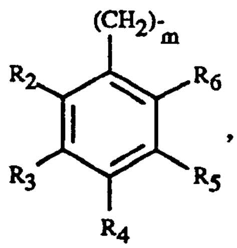
,
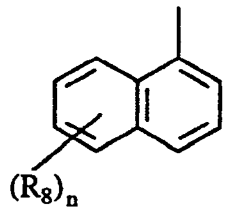
,
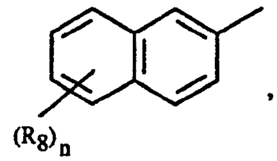
,
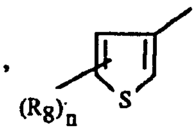
oder

(T) (U) (U') (V)

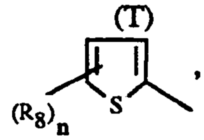
,

(V')

worin m für die Zahlen 0-4, n für die Zahlen 0-2,

$R_3$ zusammen mit $R_2$ oder mit $R_4$ für den Rest 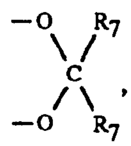 , mit $R_7$ als Wasserstoff, Fluor oder $C_1$-$C_4$-Alkyl, stehen, oder worin $R_2$-$R_6$ voneinander unabhängig für Wasserstoff, Halogen, Carboxy, $C_1$-$C_4$-Alkoxycarbonyl, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und $R_8$ für Halogen oder $C_1$-$C_4$-Alkyl stehen, bedeutet,
A den Rest

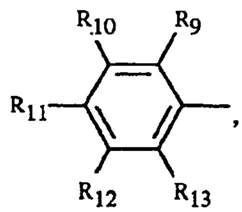
,

worin $R_{10}$ mit $R_9$ oder $R_{11}$ zusammen für den Rest 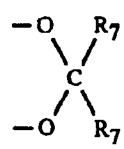 , oder worin $R_9$-$R_{13}$ voneinander unabhängig für Wasserstoff, Halogen, Carboxy, $C_1$-$C_4$-Alkoxycarbonyl,

Nitro, Cyano, die Reste 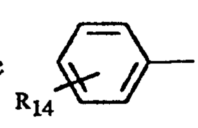 , 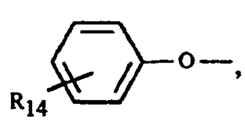 , mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet und
X -$OSO_2$, -SO- oder -$SO_2$- bedeutet, wobei wenn X den Rest -$OSO_2$-, $R_{14}$ Wasserstoff oder Chlor und $R_9$,

$R_{10}$, $R_{12}$ und $R_{13}$ Wasserstoff bedeuten, T nicht den p-Tolylrest bedeutet und wenn X den Rest $-SO_2-$ bedeutet, $R_1$ nicht den Trifluormethylrest bedeutet.

2. Verbindungen der Formel Ia, gemäss Anspruch 1

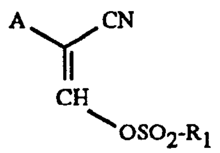

(Ia).

3. Verbindungen gemäss Anspruch 2, dadurch gekennzeichnet, dass
$R_1$ unsubstituiertes oder durch Halogen substituiertes $C_1$-$C_4$-Alkyl oder $C_5$-$C_6$-Cycloalkyl, oder die Reste T, U, U', V oder V'

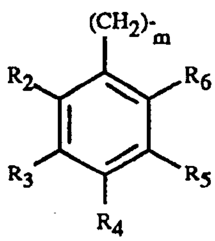 , 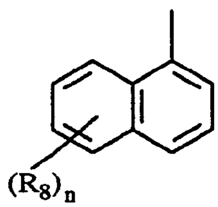 , 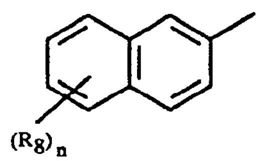 , 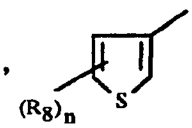 oder

(T) (U) (U') (V)

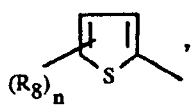 ,

(V')

worin m und n für die Zahlen 0-2,

$R_3$ zusammen mit $R_2$ oder mit $R_4$ für den Rest 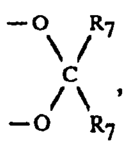 , mit $R_7$ als Wasserstoff, Fluor

stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

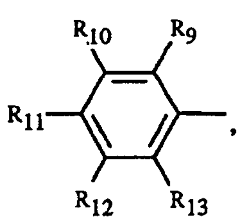 ,

worin $R_{10}$ mit $R_9$ oder $R_{11}$ zusammen für den Rest 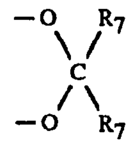, oder worin $R_9$-$R_{13}$ voneinander unabhängig für Wasserstoff, Halogen, Nitro, Cyano, die Reste 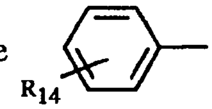, 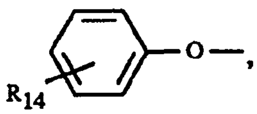, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet.

**4.** E- und Z-2-Phenyl-3-methylsulfonyloxy-acrylnitril gemäss Anspruch 3.

**5.** E- und Z-2-(4-Chlorphenyl)-3-methylsulfonyloxy-acrylnitril gemäss Anspruch 3.

**6.** E- und Z-2-(2-Tolyl)-3-methylsulfonyloxy-acrylnitril gemäss Anspruch 3.

**7.** E- und Z-2-(3-Tolyl)-3-methylsulfonyloxy-acrylnitril gemäss Anspruch 3.

**8.** Das Z-2-(3-Tolyl)-3-methylsulfonyloxy-acrylnitril gemäss Anspruch 7.

**9.** Verbindungen der Formel Ib, gemäss Anspruch 1,

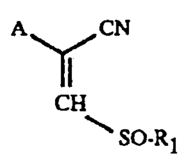

(Ib),

dadurch gekennzeichnet, dass
$R_1$ unsubstituiertes oder durch Halogen substituiertes $C_1$-$C_4$-Alkyl oder $C_5$-$C_8$-Cycloalkyl, oder die Reste T, U, U', V oder V'

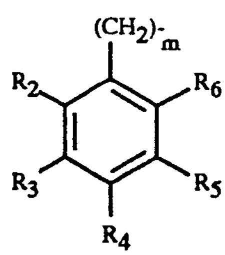, 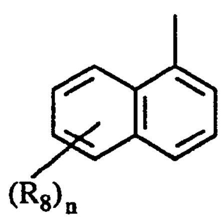, 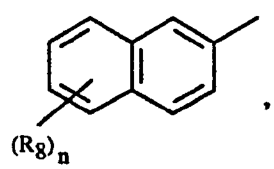, 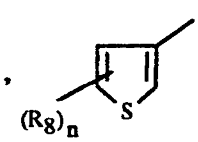 oder

(T) (U) (U') (V)

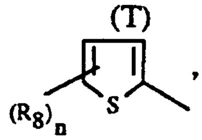,

(V')

worin m und n für die Zahlen 0-2,

$R_3$ zusammen mit $R_2$ oder mit $R_4$ für den Rest 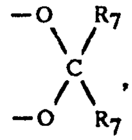 , mit $R_7$ als Wasserstoff oder

Fluor stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

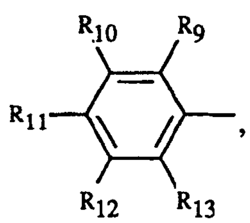

worin $R_{10}$ mit $R_9$ oder $R_{11}$ zusammen für den Rest 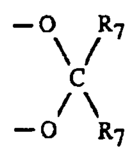 , oder worin $R_9$-$R_{13}$ voneinander unabhängig für Wasserstoff, Halogen, Nitro, Cyano, die Reste 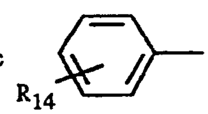 ,

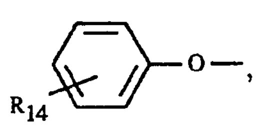 , mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet.

**10.** E- und Z-2-Phenyl-3-phenylsulfinyl-acrylnitril gemäss Anspruch 9.

**11.** E- und Z-2-Phenyl-3-methylsulfinyl-acrylnitril gemäss Anspruch 9.

**12.** Verbindungen der Formel Ic, gemäss Anspruch 1

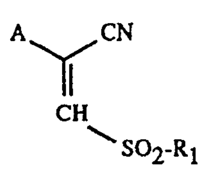

(Ic).

**13.** Verbindungen gemäss Anspruch 12, dadurch gekennzeichnet, dass
$R_1$ unsubstituiertes oder durch Halogen substituiertes $C_1$-$C_4$-Alkyl oder $C_5$-$C_8$-Cycloalkyl, oder die Reste T, U, U', V oder V'

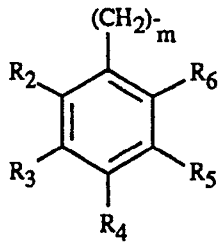, 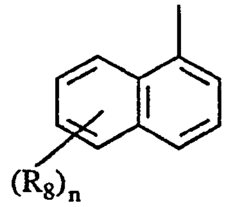, 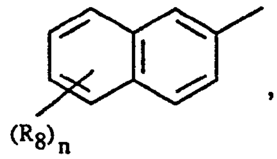, 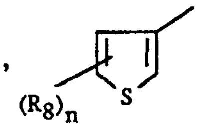 oder

(T) (U) (U') (V)

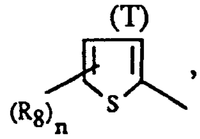,

(V')

worin m und n für die Zahlen 0-2,

$R_3$ zusammen mit $R_2$ oder mit $R_4$ für den Rest 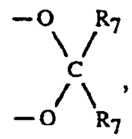, mit $R_7$ als Wasserstoff oder Fluor stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

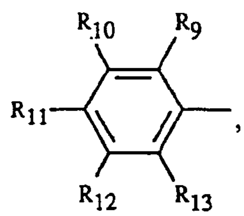,

worin $R_{10}$ mit $R_9$ oder $R_{11}$ zusammen für den Rest 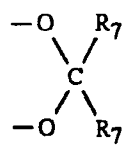, oder worin $R_9$-$R_{13}$ voneinander unabhängig für Wasserstoff, Halogen, Nitro, Cyano, die Reste 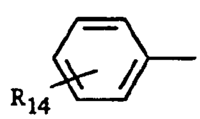, 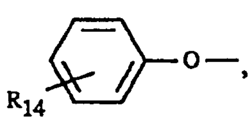, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet.

14. E- und Z-2-Phenyl-3-phenylsulfonyl-acrylnitril gemäss Anspruch 13.

15. E- und Z-2-Phenyl-3-(4-tolyl)-sulfonyl-acrylnitril gemäss Anspruch 13.

16. Z-2-Phenyl-4-(2,4-difluorphenyl)-sulfonyl-acrylnitril gemäss Anspruch 13.

17. Verfahren zur Herstellung der Verbindungen der Formel Ia, dadurch gekennzeichnet, dass man
$a_1$) ein Nitril der Formel II mit einer Base der Formel III und mit einem Ameisensäureester der Formel IV zu dem Enolat der Formel V umsetzt und letzteres mit einem Sulfochlorid der Formel VI acyliert

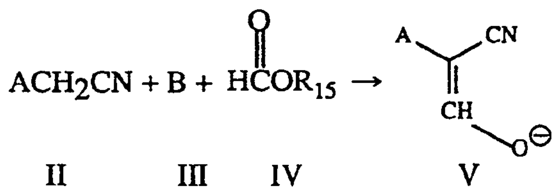

II III IV V

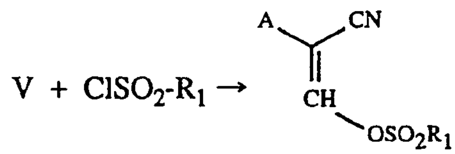

VI Ia

wobei $R_{15}$ $C_1$-$C_4$-Alkyl bedeutet, B eine Base $M^{\oplus}X^{\ominus}$, mit $M^{\oplus}$ ein Alkali- oder Erdalkalikation und mit $X^{\ominus}$ für ein Alkoholat- oder Hydrid-Anion oder für eine Aminbase, bedeutet und die restlichen Symbole die im Anspruch 1 angegebenen Bedeutungen haben, oder
$a_2$) eine Verbindung der Formel Va in Gegenwart eines säurebindenden Mittels mit einem Sulfochlorid der Formel VI acyliert

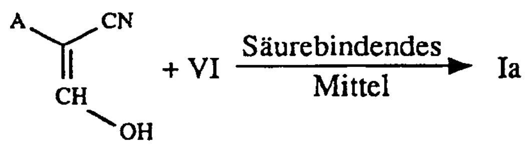


Va

und das Reaktionsprodukt isoliert, wobei A und $R_1$ die im Anspruch 1 angegebenen Bedeutungen haben.

18. Verfahren zur Herstellung der Verbindungen der Formel Ib, dadurch gekennzeichnet, dass man einen Thioether der Formel VIII mit der äquivalenten Menge eines Oxidationsmittels oxidiert

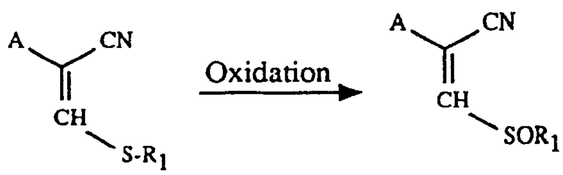


VIII Ib

und das Reaktionsprodukt isoliert.

19. Verfahren zur Herstellung der Verbindungen der Formel Ic, dadurch gekennzeichnet, dass man
$c_1$) eine Verbindung der Formel Ia gemäss Anspruch 17, mit einem Sulfinsäuresalz der Formel VII, worin $M^{\oplus}$ die im Anspruch 17 und $R_1$ die in Anspruch 1 angegebenen Bedeutungen haben

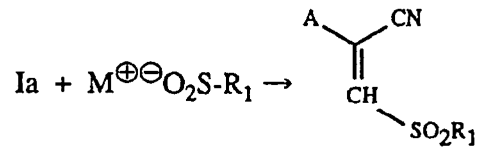


VII Ia

umsetzt oder

$c_2$) ein Sulfoxid der Formel Ib, Anspruch 18 oxidiert

$$\mathrm{Ib} \xrightarrow{\text{Oxidation}} \mathrm{Ic}$$

und das Reaktionsprodukt isoliert.

**20.** Mikrobizide Mittel enthaltend als mindestens einen Wirkstoff eine Verbindung der Formel I

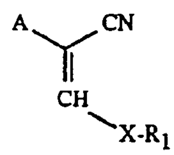

(I),

welche als Gemische (E,Z) oder als reine E- und Z-Isomeren vorliegen, worin $R_1$ unsubstituiertes oder durch Halogen, $C_1$-$C_4$-Alkoxy substituiertes $C_1$-$C_6$-Alkyl oder $C_5$-$C_7$-Cycloalkyl, oder die Reste T, U, U', V oder V'

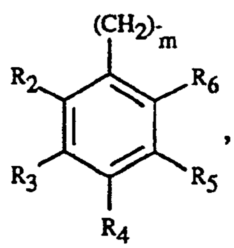 , 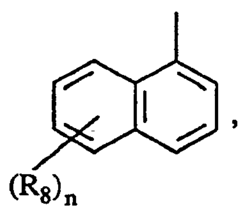 , 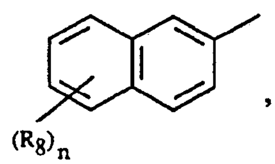 , 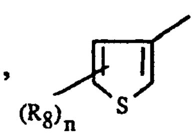 oder

(T) (U) (U') (V)

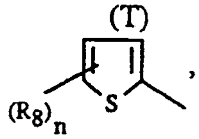 ,

(V')

worin m für die Zahlen 0-4, n für die Zahlen 0-2,

$R_3$ zusammen mit $R_2$ oder mit $R_4$ für den Rest 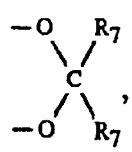 , mit $R_7$ als Wasserstoff, Fluor oder $C_1$-$C_4$-Alkyl, stehen, oder worin $R_2$-$R_6$ voneinander unabhängig für Wasserstoff, Halogen, Carboxy, $C_1$-$C_4$-Alkoxycarbonyl, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und $R_8$ für Halogen oder $C_1$-$C_4$-Alkyl stehen, bedeutet,

A den Rest

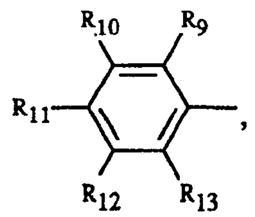

worin $R_{10}$ mit $R_9$ oder $R_{11}$ zusammen für den Rest 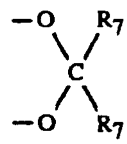, oder worin $R_9$-$R_{13}$ voneinander unabhängig für Wasserstoff, Halogen, Carboxy, $C_1$-$C_4$-Alkoxycarbonyl, Nitro, Cyano, die Reste 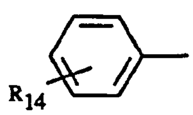, 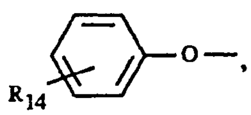, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet und X -$OSO_2$-, -SO- oder -$SO_2$- bedeutet.

21. Mittel gemäss Anspruch 20 enthaltend als Wirkstoff eine Verbindung der Formel I gemäss Anspruch 1.

22. Verwendung von Verbindungen der Formel I gemäss Anspruch 20 zur Bekämpfung oder Verhütung eines Befalls von phytopathogenen Mikroorganismen.

23. Verfaren zur Bekämpfung oder Verhütung eines Befalls von Pflanzen durch phytopathogene Mikroorganismen, dadurch gekennzeichnet, dass man eine Verbindung der Formel I gemäss Anspruch 20 auf die Pflanze, Pflanzenteile oder deren Standort appliziert.

24. Verfaren gemäss Anspruch 23, wobei die Pflanzenteile das Saatgut sind.

**Patentansprüche Für folgenden Vertragsstaat : ES**

1. Mikrobizide Mittel enthaltend als mindestens einen Wirkstoff eine Verbindung der Formel I

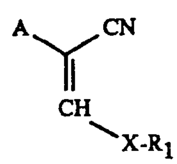

(I),

welche als Gemische (E,Z) oder als reine E- und Z-Isomeren vorliegen, worin $R_1$ unsubstituiertes oder durch Halogen, $C_1$-$C_4$-Alkoxy substituiertes $C_1$-$C_6$-Alkyl oder $C_5$-$C_7$-Cycloalkyl, oder die Reste T, U, U', V oder V'

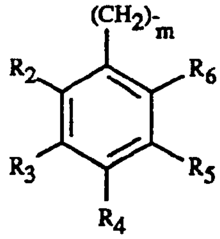, 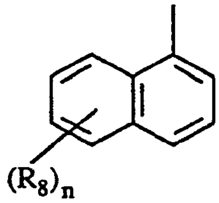, 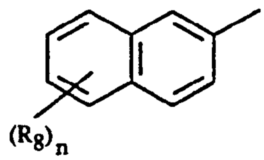, 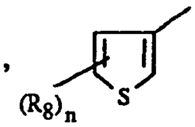 oder

(T) (U) (U') (V)

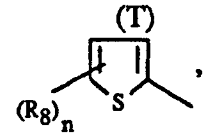,

(V')

worin m für die Zahlen 0-4, n für die Zahlen 0-2,

$R_3$ zusammen mit $R_2$ oder mit $R_4$ für den Rest 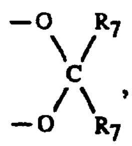, mit $R_7$ als Wasserstoff, Fluor oder $C_1$-$C_4$-Alkyl, stehen, oder worin $R_2$-$R_6$ voneinander unabhängig für Wasserstoff, Halogen, Carboxy, $C_1$-$C_4$-Alkoxycarbonyl, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

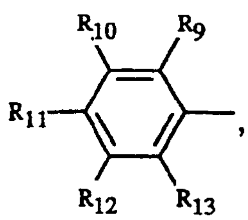,

worin $R_{10}$ mit $R_9$ oder $R_{11}$ zusammen für den Rest 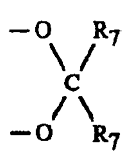, oder worin $R_9$-$R_{13}$ voneinander unabhängig für Wasserstoff, Halogen, Carboxy, $C_1$-$C_4$-Alkoxycarbonyl,

Nitro, Cyano, die Reste 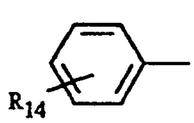, 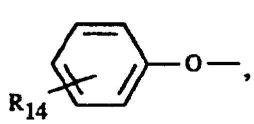, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet und
X $-OSO_2-$, -SO- oder $-SO_2-$ bedeutet.

2. Mittel gemäss Anspruch 1 enthaltend als Wirkstoff eine Verbindung der Formel I, wobei wenn X den Rest $-OSO_2-$, $R_{14}$ Wasserstoff oder Chlor und $R_9$, $R_{10}$, $R_{12}$ und $R_{13}$ Wasserstoff bedeuten, T nicht den p-Tolylrest

bedeutet und wenn X den Rest $-SO_2-$ bedeutet, $R_1$ nicht den Trifluormethylrest bedeutet.

3. Mittel gemäss Anspruch 2 enthaltend als Wirkstoff eine Verbindung der Formel Ia

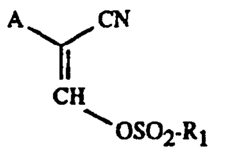 (Ia).

4. Mittel gemäss Anspruch 3, dadurch gekennzeichnet, dass $R_1$ unsubstituiertes oder durch Halogen substituiertes $C_1$-$C_4$-Alkyl oder $C_5$-$C_8$-Cycloalkyl, oder die Reste T, U, U', V oder V'

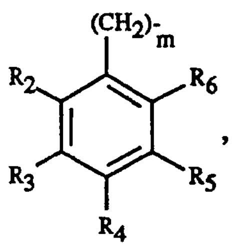 , 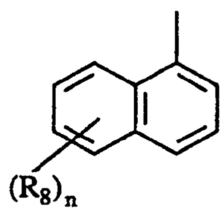 , 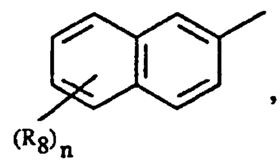 , 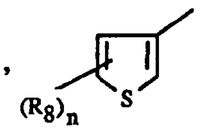 oder

(T) (U) (U') (V)

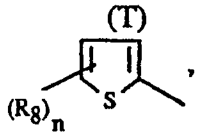 ,

(V')

worin m und n für die Zahlen 0-2,

$R_3$ zusammen mit $R_2$ oder mit $R_4$ für den Rest 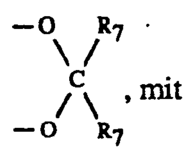 , mit $R_7$ als Wasserstoff, Fluor

stehen, oder worin $R_2$-$R_8$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

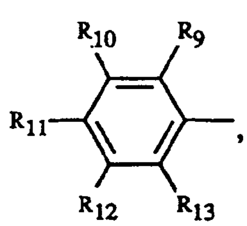 ,

worin $R_{10}$ mit $R_9$ oder $R_{11}$ zusammen für den Rest 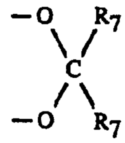 , oder worin $R_9$-$R_{13}$ voneinander unabhängig für Wasserstoff, Halogen, Nitro, Cyano, die Reste 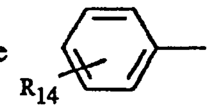, 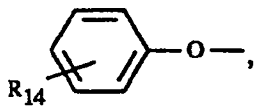, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet.

5. Mittel gemäss Anspruch 4, dadurch gekennzeichnet, dass es als Wirkstoff E- und Z-2-Phenyl-3-methylsulfonyloxy-acrylnitril enthält.

6. Mittel gemäss Anspruch 4, dadurch gekennzeichnet, dass es als Wirkstoff E- und Z-2-(4-Chlorphenyl)-3-methylsulfonyloxy-acrylnitril enthält.

7. Mittel gemäss Anspruch 4, dadurch gekennzeichnet, dass es als Wirkstoff E- und Z-2-(2-Tolyl)-3-methylsulfonyloxy-acrylnitril enthält.

8. Mittel gemäss Anspruch 4, dadurch gekennzeichnet, dass es als Wirkstoff E- und Z-2- (3-Tolyl)-3-methylsulfonyloxy-acrylnitril enthält.

9. Mittel gemäss Anspruch 8, dadurch gekennzeichnet, dass es als Wirkstoff Z-2-(3-Tolyl)-3-methylsulfonyloxy-acrylnitril enthält.

10. Mittel gemäss Anspruch 2 enthaltend als Wirkstoff eine Verbindung der Formel Ib

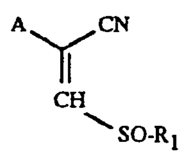

(Ib),

worin
$R_1$ unsubstituiertes oder durch Halogen substituiertes $C_1$-$C_4$-Alkyl oder $C_5$-$C_6$-Cycloalkyl, oder die Reste T, U, U', V oder V'

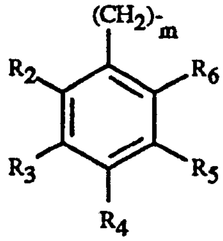 , 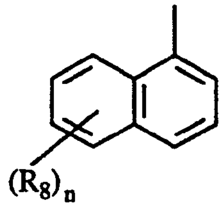 , 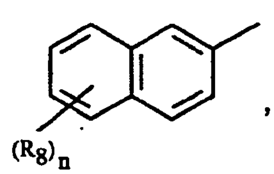 , 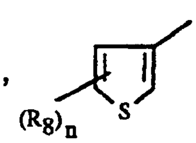 oder

(T) (U) (U') (V)

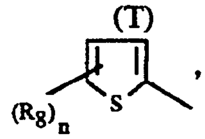 ,

(V')

worin m und n für die Zahlen 0-2,

$R_3$ zusammen mit $R_2$ oder mit $R_4$ für den Rest 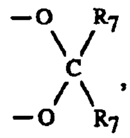 , mit $R_7$ als Wasserstoff oder Fluor stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und
$R_8$ für Halogen oder $C_1$-$C_4$-Alkyl stehen, bedeutet,
A den Rest

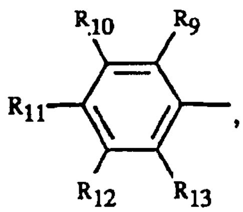,

worin $R_{10}$ mit $R_9$ oder $R_{11}$ zusammen für den Rest 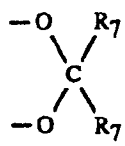 , oder worin $R_9$-$R_{13}$ voneinander unabhängig für Wasserstoff, Halogen, Nitro, Cyano, die Reste 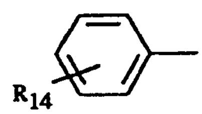,

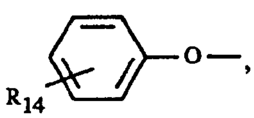, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet.

11. Mittel gemäss Anspruch 10, dadurch gekennzeichnet, dass es als Wirkstoff E- und Z-2-Phenyl-3-phenylsulfinyl-acrylnitril enthält.

12. Mittel gemäss Anspruch 10, dadurch gekennzeichnet, dass es als Wirkstoff E- und Z-2-Phenyl-3-methylsulfinyl-acrylnitril enthält.

13. Mittel gemäss Anspruch 2 enthaltend als Wirkstoff eine Verbindung der Formel Ic

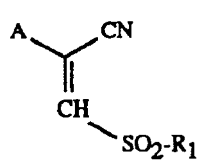

(Ic).

14. Mittel gemäss Anspruch 13, dadurch gekennzeichnet, dass
$R_1$ unsubstituiertes oder durch Halogen substituiertes $C_1$-$C_4$-Alkyl oder $C_5$-$C_6$-Cycloalkyl, oder die Reste T, U, U', V oder V'

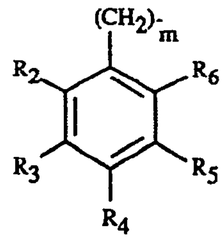, 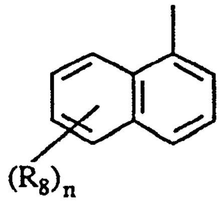, 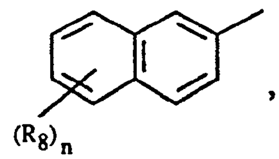, 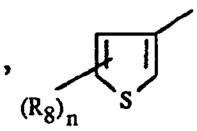 oder

(T) (U) (U') (V)

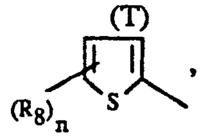,

(V')

worin m und n für die Zahlen 0-2,

$R_3$ zusammen mit $R_2$ oder mit $R_4$ für den Rest 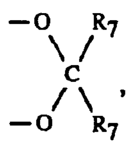, mit $R_7$ als Wasserstoff oder Fluor stehen, oder worin $R_2$-$R_6$ voneinander unabhängig für Wasserstoff, Halogen, Nitro, Cyano, oder für die unsubstituierten oder durch Halogen substituierten Reste $C_1$-$C_4$-Alkyl, Phenyl, $C_1$-$C_4$Alkoxy, $C_1$-$C_4$-Alkylthio und

$R_8$ für Halogen oder $C_1$-$C_4$-Alkyl stehen, bedeutet,

A den Rest

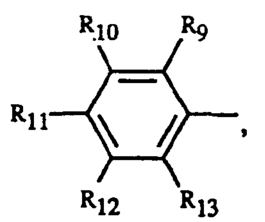,

worin $R_{10}$ mit $R_9$ oder $R_{11}$ zusammen für den Rest 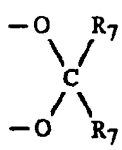, oder worin $R_9$-$R_{13}$ voneinander unabhängig für Wasserstoff, Halogen, Nitro, Cyano, die Reste 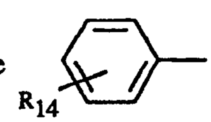, 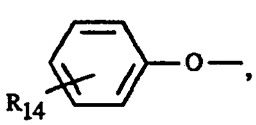, mit $R_{14}$ als Wasserstoff, Halogen oder $C_1$-$C_4$-Alkyl oder für die unsubstituierten oder durch Halogen substituierten Reste $C_1$-$C_4$-Alkyl, $C_1$-$C_4$-Alkoxy oder $C_1$-$C_4$-Alkylthio stehen, oder die Reste U, U', V und V' bedeutet.

**15.** Mittel gemäss Anspruch 14, dadurch gekennzeichnet, dass es als Wirkstoff E- und Z-2-Phenyl-3-phenylsulfonyl-acrylnitril enthält.

16. Mittel gemäss Anspruch 14, dadurch gekennzeichnet, dass es als Wirkstoff E- und Z-2-Phenyl-3-(4-tolyl)-sulfonyl-acrylnitril enthält.

17. Mittel gemäss Anspruch 14, dadurch gekennzeichnet, dass es als Wirkstoff Z-2-Phenyl-4-(2,4-difluorphenyl)-sulfonyl-acrylnitril enthält.

18. Verfahren zur Herstellung der Verbindungen der Formel Ia, dadurch gekennzeichnet, dass man
$a_1$) ein Nitril der Formel II mit einer Base der Formel III und mit einem Ameisensäureester der Formel IV zu dem Enolat der Formel V umsetzt und letzteres mit einem Sulfochlorid der Formel VI acyliert

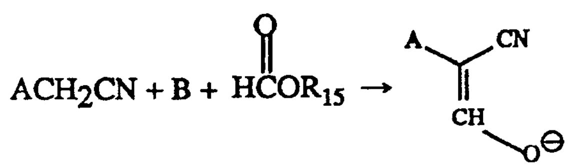

II III IV V

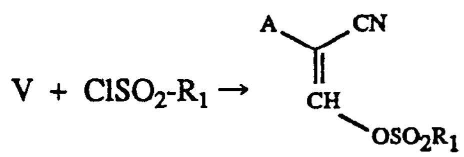

VI Ia

wobei $R_{15}$ $C_1$-$C_4$-Alkyl bedeutet, B eine Base $M^{\oplus}X^{\ominus}$, mit $M^{\oplus}$ ein Alkali- oder Erdalkalikation und mit $X^{\ominus}$ für ein Alkoholat- oder Hydrid-Anion oder für eine Aminbase, bedeutet und die restlichen Symbole die im Anspruch 1 angegebenen Bedeutungen haben, oder
$a_2$) eine Verbindung der Formel Va in Gegenwart eines säurebindenden Mittels mit einem Sulfochlorid der Formel VI acyliert

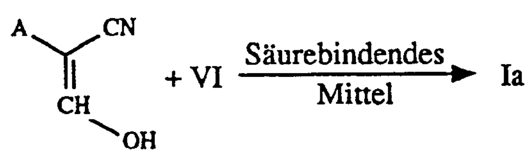


Va

und das Reaktionsprodukt isoliert, wobei A und $R_1$ die im Anspruch 1 angegebenen Bedeutungen haben.

19. Verfahren zur Herstellung der Verbindungen der Formel Ib, dadurch gekennzeichnet, dass man einen Thioether der Formel VIII mit der äquivalenten Menge eines Oxidationsmittels oxidiert

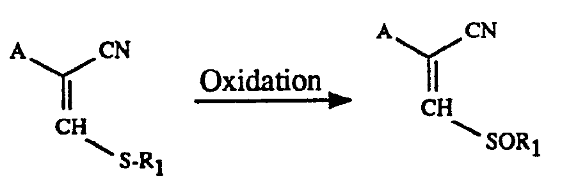


VIII Ib

und das Reaktionsprodukt isoliert.

**20.** Verfahren zur Herstellung der Verbindungen der Formel Ic, dadurch gekennzeichnet, dass man $c_1$) eine Verbindung der Formel Ia gemäss Anspruch 18, mit einem Sulfinsäuresalz der Formel VII, worin $M^{\oplus}$ die im Anspruch 18 und $R_1$ die in Anspruch 2 angegebenen Bedeutungen haben

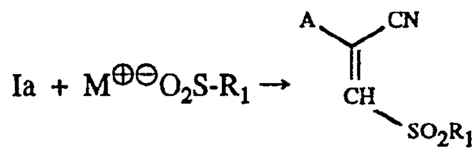

VII Ia

umsetzt oder

$c_2$) ein Sulfoxid der Formel Ib, Anspruch 19 oxidiert

$$\text{Ib} \xrightarrow{\text{Oxidation}} \text{Ic}$$

und das Reaktionsprodukt isoliert.

**21.** Verfahren zur Verwendung von Mitteln gemäss Anspruch 1 zur Bekämpfung oder Verhütung eines Befalls von phytopathogenen Mikroorganismen.

**22.** Verfahren zur Bekämpfung oder Verhütung eines Befalls von Pflanzen durch phytopathogene Mikroorganismen, dadurch gekennzeichnet, dass man ein Mittel der Formel I gemäss Anspruch 21 auf die Pflanze, Pflanzenteile oder deren Standort appliziert.

**23.** Verfahren gemäss Anspruch 22, wobei die Pflanzenteile das Saatgut sind.

Europäisches Patentamt

# EUROPÄISCHER RECHERCHENBERICHT

Nummer der Anmeldung

EP 91810021.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| A | US - A - 4 388 249 (ARNOLD D. GUTMAN et al.) * Zusammenfassung * -- | 1,12, 20 | C 07 C 317/44<br>C 07 C 309/63<br>C 07 D 333/24<br>C 07 D 317/60<br>A 01 N 41/02 |
| A | EP - A1 - 0 051 198 (BAYER) * Zusammenfassung * -- | 1,12, 20 | |
| A | GB - A - 1 249 087 (FARBENFABRIKEN BAYER) * Ansprüche 1,30 * ---- | 1,2,20 | |

RECHERCHIERTE SACHGEBIETE (Int Cl.⁵)

C 07 C 317/00
C 07 C 309/00
C 07 C 323/00
C 07 D 333/00
C 07 D 317/00

Der vorliegende Recherchenbericht wurde für alle Patentansprüche erstellt.

| Recherchenort | Abschlußdatum der Recherche | Prüfer |
|---|---|---|
| WIEN | 24-04-1991 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62